# EUROPEAN PATENT APPLICATION

(11) **EP 4 755 395 A1**
(43) Date of publication of application: **10.06.2026**
(21) Application number: 24849092.2
(22) Date of filing: 26.07.2024
(51) Int. Cl.: A61K 38/38, A61K 33/30, A61P 1/16, A61P 43/00

(54) **PHARMACEUTICAL COMPOSITION FOR INTRAVENOUS INJECTION**

(30) Priority: 28.07.2023 JP 2023123891
(71) Applicant: Otsuka Pharmaceutical Factory, Inc., Naruto-shi, Tokushima 772-8601 (JP)
(72) Inventor: TANAKA, Makoto, Naruto-shi, Tokushima 772-8601 (JP)
(74) Representative: Hoffmann Eitle
(86) International application number: PCT/JP2024/026793
(87) International publication number: WO 2025/028439

(57) **Abstract**

The primary purpose of the present invention is to provide, for albumin deficiency symptoms which require administration of zinc, a formulation that is capable of further improving the efficiency of zinc replenishment. The secondary purpose of the present invention is to provide a formulation that has a treatment effect for iron overload disorders. **In** the present invention, a first pharmaceutical composition, for intravenous injection and containing zinc ions and serum albumin, is capable of more efficiently replenishing zinc for albumin deficiency symptoms that require administration of zinc, and a second composition, for intravenous injection and containing zinc ions, is capable of removing iron in vivo for iron overload disorders.

## Description

### Technical Field

The present invention relates to an intravenous pharmaceutical composition that efficiently supplies zinc for intercurrent conditions of hypoalbuminemia and hypozincemia, and an intravenous pharmaceutical composition that has an effect of removing iron in the body for iron overload.

### Background Art

Zinc plays an important role in many physiological functions and activities and is one of important nutrients for humans. When zinc is deficient, protein synthesis in the body is reduced, and damage easily occurs in organs and cells in which protein synthesis is active. For example, dysgeusia, dermatitis, alopecia, anemia, stomatitis, male sexual dysfunction, susceptibility to infection, osteoporosis, and the like are likely to occur due to zinc deficiency. Furthermore, zinc enzymes play important roles in nitrogen and ammonia metabolism in the liver.

Several factors for zinc deficiency have been reported. For example, NPL 1 reports that the amount of zinc storage in hepatocytes decreases by 70% with the lack of albumin, NPL 2 reports that albumin is relevant as a factor associated with hypozincemia in dialysis patients, NPL 3 reports that albumin is involved in the blood zinc concentration in patients with liver cirrhosis, and NPL 4 speculates that zinc is likely to bind to an amino acid due to the decrease in albumin in liver diseases, resulting in an increase in urinary excretion of zinc and a decrease in blood concentration of zinc.

Furthermore, NPL 5 describes the administration of zinc acetate to patients with liver cirrhosis having zinc deficiency, and NPL 6 describes the administration of zinc acetate to patients with liver cirrhosis accompanied by hyperammonemia and hypozincemia. As described in NPL 5, the treatment effect of the administration of zinc for hyperammonemia has been reported in a small number of patients with liver cirrhosis. On the other hand, in the systematic review in NPL 7, it has been reported that serum ammonia in patients with liver cirrhosis is not improved by the administration of zinc acetate or zinc sulfate.

Similarly to zinc, iron is one of the essential trace elements, and iron itself or iron as a component of heme and an iron-sulfur complex are important nutrients in maintaining homeostasis of the body. While the excretion route for iron does not exist in the body, which is a reasonable physiological characteristic that allows iron as the essential trace element to be secured, it suggests that the body easily falls into an iron overload condition. It has been reported that iron circulates in the body by binding to transferrin, which is a glycoprotein, whereas in the disease state of iron overload, non-transferrin-bound iron (NTBI) is increased which plays a major role in the pathogenesis of various diseases by catalyzing the Fenton reaction, thus generating hydroxyl radicals. Clinically, iron overload occurs due to mutations in iron-related genes, continuous transfusion, and administration of an intravenous iron preparation.

A drug therapy for iron overload is carried out by iron chelation therapy. Deferoxamine was used as an iron chelating agent, but it was difficult to use deferoxamine in outpatients, since the half-life thereof was short, and continuous injection was required to obtain the effect. Thereafter, deferasirox, which is an oral preparation with a long half-life, has become available, but it has been reported in NPL 8 that since specific adverse events such as visual impairment, auditory impairment, renal dysfunction, hepatic dysfunction, and gastrointestinal symptoms have been observed with these iron chelating agents, it is necessary to regularly monitor such adverse events in addition to an iron dynamics marker indicating the treatment effect.

As basic knowledge on the interaction of iron and zinc in the body and the biological absorption of both elements, it has been reported in NPL 9 that Zip 14, a transporter present on the cell membrane, plays a role in zinc and iron metabolism by mediating the uptake of zinc and NTBI into cells, and the uptake of iron in Zip 14 is inhibited by zinc. In addition, as findings also clinically supporting this absorption antagonistic effect, NPL 10 reports the relationship between hypozincemia and iron overload, specifically describing that the serum zinc concentration in patients with chronic liver disease inversely correlates with the serum ferritin concentration which reflects the amount of iron storage in tissues, and that zinc deficiency promotes iron overload and ultimately induces fatty liver by lipid peroxidation.

### Citation List

### Non Patent Literature

NPL 1: Zinc uptake by isolated rat liver parenchymal cells, Biochimica et Ciophysica Acta, 538 (1978) 435-444
NPL 2: Serum zinc concentration distribution in dialysis patients -Factors associated with hypozincemia- Journal of Japanese Society for Dialysis Therapy 51(6): 369-377, 2018
NPL 3: Significance of blood loss zinc concentration measurement in treatment strategy for liver cirrhosis, Liver 62, Suppl. (1), 2021, Mini Oral, The 57th Annual Meeting of the Japan Society of Hepatology, SY4-8
NPL 4: Ammonia metabolism and hepatic encephalopathy. Hepatology Research, 2004, 30: 73-80
NPL 5: Recent findings on zinc deficiency and chronic liver disease -Focusing on personal case studies-, Journal of Zinc Nutritional Therapy 10(2), 2020, 54-63
NPL 6: Effect of zinc on liver cirrhosis with hyperammonemia: A preliminary randomized, placebo-controlled double-blind trial, Nutrition 30 (2014) 1409-1414
NPL 7: Zinc supplementation in patients with cirrhosis and hepatic encephalopathy: a systematic review and meta-analysis, Shen et al. Nutrition Journal (2019) 18:34
NPL 8: Reference guide for treatment of post-transfusion iron overload, Revised in 2019.
NPL 9: Zip14 (Slc39a14) mediates non-transferrin-bound iron uptake into cells. Juan P. Liuzzi, Fikret Aydemir, Hyeyoung Nam, Mitchell D. Knutson, and Robert J. Cousins
NPL 10: Exacerbation of Insulin Resistance and Hepatic Steatosis Deriving from Zinc Deficiency in Patients with HCV-Related Chronic Liver Disease, Takashi Himoto.

### Summary of Invention

### Technical Problem

The liver plays a central role in short-term zinc homeostasis regulation and zinc storage. For a condition of insufficient zinc storage in the liver, intravenous injection of a zinc salt is expected to increase the serum zinc concentration and increase zinc in the liver in association with the normalization of the serum zinc level. For example, for a condition in which both albumin deficiency and zinc deficiency occur, a certain level of treatment effect can be expected by administering a zinc preparation as described in NPL 4. However, the effect is limited, and there is still room for improvement. In addition, for the treatment of hyperammonemia in patients with liver cirrhosis accompanied by albumin deficiency, administration of a zinc preparation as described in PTL 5 and 6 can be expected to have a certain level of treatment effect. However, the effect is limited, and there is still room for improvement.

Furthermore, since continuous injection is required in a drug therapy for iron overload, and the drug therapy is associated with adverse events as described in NPL 7, there are problems such as the physical restraint at the time of administration and restriction of administration in patients having specific backgrounds, respectively.

Therefore, a primary object of the present invention is to provide, for an albumin deficiency condition which requires administration of zinc, a formulation that is capable of further improving the efficiency of zinc supplementation.

A secondary object of the present invention is to provide a new formulation which has an effect of removing iron in the body for iron overload and can be conveniently used without using an iron chelating agent associated with specific adverse events.

### Solution to Problem

As a result of intensive studies, the present inventors have unexpectedly found that, first, the efficiency of zinc supplementation is improved by intravenously injecting a pharmaceutical composition of a new formulation containing both zinc and serum albumin, and second, for iron overload, an effect of removing iron in the body which is comparable to that of known iron overload is obtained by intravenously injecting zinc. The present invention has been completed by further conducting studies based on these findings.

That is, the present invention provides inventions of the following aspects (a first intravenous pharmaceutical composition set forth in items 1 to 11 and a second intravenous pharmaceutical composition set forth in items 12 to 23).
Item 1. An intravenous pharmaceutical composition including: a zinc ion; and serum albumin.
Item 2. The intravenous pharmaceutical composition according to item 1, in which the zinc ion and the serum albumin form a conjugate.
Item 3. The intravenous pharmaceutical composition according to item 1 or 2, in which a source of the zinc ion is a water-soluble zinc salt.
Item 4. The intravenous pharmaceutical composition according to item 3, in which the water-soluble zinc salt is zinc sulfate.
Item 5. The intravenous pharmaceutical composition according to any one of items 1 to 4, in which the content of the serum albumin per 1 mol of the zinc ion is 0.35 mol or more.
Item 6. The intravenous pharmaceutical composition according to any one of items 1 to 5, in which the content of the zinc ion at time of use is 0.1 mM or more.
Item 7. The intravenous pharmaceutical composition according to any one of items 1 to 6, in which the intravenous pharmaceutical composition is free of a carbohydrate.
Item 8. The intravenous pharmaceutical composition according to any one of items 1 to 7, in which the intravenous pharmaceutical composition is an injection.
Item 9. The intravenous pharmaceutical composition according to any one of items 1 to 8, in which the intravenous pharmaceutical composition is applied for hypoalbuminemia that is a condition requiring administration of zinc.
Item 10. The intravenous pharmaceutical composition according to item 9, in which the condition is hypoalbuminemic hypozincemia.
Item 11. The intravenous pharmaceutical composition according to item 9 or 10, in which the intravenous pharmaceutical composition is applied for hyperammonemia.
Item 12. An intravenous pharmaceutical composition including a zinc ion, in which the intravenous pharmaceutical composition is used for treatment of iron overload.
Item 13. The intravenous pharmaceutical composition according to item 12, in which a source of the zinc ion is a water-soluble zinc salt.
Item 14. The intravenous pharmaceutical composition according to item 13, in which the water-soluble zinc salt is zinc sulfate.
Item 15. The intravenous pharmaceutical composition according to any one of items 12 to 14, in which the content of the zinc ion at time of use is 0.1 mM or more.
Item 16. The intravenous pharmaceutical composition according to any one of items 12 to 15, in which the intravenous pharmaceutical composition is an injection.
Item 17. The intravenous pharmaceutical composition according to any one of items 12 to 16, further including serum albumin.
Item 18. The intravenous pharmaceutical composition according to item 17, in which the zinc ion and the serum albumin form a conjugate.
Item 19. The intravenous pharmaceutical composition according to item 17 or 18, in which the content of the serum albumin per 1 mol of the zinc ion is 0.35 mol or more.
Item 20. The intravenous pharmaceutical composition according to any one of items 17 to 19, in which the iron overload is accompanied by hypoalbuminemia.
Item 21. The intravenous pharmaceutical composition according to item 20, in which the hypoalbuminemia is hypoalbuminemic hypozincemia.
Item 22. The intravenous pharmaceutical composition according to any one of items 12 to 21, in which the iron overload is accompanied by hyperammonemia.
Item 23. The intravenous pharmaceutical composition according to any one of items 12 to 22, in which the intravenous pharmaceutical composition is free of a carbohydrate.
Item 24. A method for supplementing zinc, the method including a step of intravenously injecting a pharmaceutical composition to a subject with hypoalbuminemia requiring administration of zinc, the pharmaceutical composition including therapeutically effective amounts of a zinc ion and serum albumin.
Item 25. Use of an intravenous pharmaceutical composition for production of a zinc supplement for hypoalbuminemic hypozincemia, the intravenous pharmaceutical composition including a zinc ion and serum albumin.
Item 26. An intravenous pharmaceutical composition including a zinc ion and serum albumin, in which the intravenous pharmaceutical composition is for use in zinc supplementation in hypoalbuminemic hypozincemia.
Item 27. A method for treating iron overload, the method including intravenously injecting an effective amount of a zinc ion to a subject in need of treatment of iron overload.
Item 28. Use of a zinc ion for production of an intravenous pharmaceutical composition for treating iron overload.
Item 29. A zinc ion for use in treatment of iron overload.

### Advantageous Effects of Invention

According to the present invention, first, there is provided a formulation capable of further improving the efficiency of zinc supplementation for hypoalbuminemia which is a condition requiring administration of zinc, and second, there is provided a formulation with which an effect of removing iron in the body is obtained for iron overload.

### Brief Description of Drawings

[Fig. 1] Fig. 1 shows the concentrations of a zinc ion bound to albumin in intravenous solutions obtained by mixing different concentrations of albumin to 1 mM zinc sulfate (Test Example 1-1).
[Fig. 2] Fig. 2 shows the results of comparing the binding strength of zinc ions to albumin between intravenous solutions containing zinc sulfate and albumin and intravenous solutions containing zinc acetate or zinc chloride and albumin (Test Example 1-2).
[Fig. 3A] Fig. 3A shows the serum zinc concentrations in the case of single administration of a first intravenous pharmaceutical composition of the present invention (Zn-Alb) to model rats of hypoalbuminemic hypozincemia.
[Fig. 3B] Fig. 3B shows the increased amounts of serum zinc in the case of the single administration of the first intravenous pharmaceutical composition of the present invention (Zn-Alb) to the model rats of hypoalbuminemic hypozincemia.
[Fig. 4A] Fig. 4A shows the serum zinc concentrations in the case of repeated administration of the first intravenous pharmaceutical composition of the present invention (Zn-Alb) to model rats of hypoalbuminemic hypozincemia.
[Fig. 4B] Fig. 4B shows the increased amounts of serum zinc in the case of the repeated administration of the first intravenous pharmaceutical composition of the present invention (Zn-Alb) to the model rats of hypoalbuminemic hypozincemia.
[Fig. 5A] Fig. 5A shows the serum ammonia reduction effect in the case of repeated administration of the first intravenous pharmaceutical composition of the present invention (Zn-Alb 200, 400, and 600) to model rats of hypoalbuminemic hypozincemia accompanied by hyperammonemia.
[Fig. 5B] Fig. 5B shows the liver zinc increasing effect in the case of the repeated administration of the first intravenous pharmaceutical composition of the present invention (Zn-Alb 200, 400, and 600) to the model rats of hypoalbuminemic hypozincemia.
[Fig. 6A] Fig. 6A shows comparison of the plasma ferritin reduction effect between a second intravenous pharmaceutical composition of the present invention (Zn and Zn-Alb) and deferasirox (therapeutic agent for iron overload).
[Fig. 6B] Fig. 6B shows comparison of the plasma iron reduction effect between the second intravenous pharmaceutical composition of the present invention (Zn and Zn-Alb) and deferasirox (therapeutic agent for iron overload).
[Fig. 6C] Fig. 6C shows comparison of the unsaturated iron binding capacity (UIBC) increasing effect between the second intravenous pharmaceutical composition of the present invention (Zn and Zn-Alb) and deferasirox (therapeutic agent for iron overload).
[Fig. 7] Fig. 7 shows the serum iron reduction effect in the case of repeated administration of the second intravenous pharmaceutical composition of the present invention (Zn-Sul and Zn-Alb) to iron overload model rats.
[Fig. 8] Fig. 8 shows the serum iron reduction effect in the case of repeated administration of the second intravenous pharmaceutical composition of the present invention (Zn and Zn-Alb 200 and 600) to model rats of iron overload accompanied by hypoalbuminemic hypozincemia.

### Description of Embodiments

### 1. First Intravenous Pharmaceutical Composition

A first intravenous pharmaceutical composition of the present invention includes a zinc ion and serum albumin. Hereinafter, the first intravenous pharmaceutical composition of the present invention will be described in detail.

### Source of Zinc Ion

As the source of the zinc ion, a water-soluble zinc salt can be used. The water-soluble zinc salt is not particularly limited as long as it is a pharmaceutically acceptable salt. Examples of the water-soluble zinc salt include salts of inorganic acids such as hydrochloric acid, hydrobromic acid, phosphoric acid, and sulfuric acid; and salts of organic acids such as acetic acid, citric acid, tartaric acid, lactic acid, succinic acid, fumaric acid, maleic acid, and methanesulfonic acid. The water-soluble zinc salt may be an anhydrous form or a hydrated form.

As the water-soluble zinc salt in the first intravenous pharmaceutical composition of the present invention, one of the above salts may be used singly, or two or more thereof may be used in combination. Among the above salts, salts of inorganic acid salts are preferable, a sulfate (that is, zinc sulfate) is more preferable, and zinc sulfate heptahydrate is still more preferable from the viewpoint of further improving the zinc supplementation efficiency.

The content of the zinc ion may be appropriately set according to the required degree of improvement in the zinc supplementation efficiency, and/or the necessity of dilution and dissolution at time of use, and the like. For example, the zinc ion concentration in the intravenous pharmaceutical composition at time of use is, for example, 0.1 mM or more, preferably 0.5 mM or more, more preferably 0.7 mM or more, still more preferably 0.8 mM or more, and even more preferably 0.9 mM or more. The upper limit of the zinc ion concentration is not particularly limited and is, for example, 5 mM or less, preferably 4 mM or less, more preferably 3 mM or less, still more preferably 2 mM or less, even more preferably 1.5 mM or less, and yet even more preferably 1.2 mM or less. A specific range of the zinc ion concentration in the intravenous pharmaceutical composition at time of use is, for example, 0.1 to 5 mM or 0.1 to 4 mM, preferably 0.5 to 3 mM, more preferably 0.7 to 2 mM, still more preferably 0.8 to 1.5 mM, and even more preferably 0.9 to 1.2 mM.

### Serum Albumin

The origin of the serum albumin is not particularly limited as long as it is common to the animal species to which the intravenous pharmaceutical composition is to be administered.

The content of the serum albumin may be appropriately set according to the required degree of improvement in the zinc supplementation efficiency, and the content of the serum albumin per 1 mol of the zinc ion is, for example, 0.35 mol or more, preferably 0.4 mol or more, and more preferably 0.5 mol or more or 0.55 mol or more. The upper limit of the content of the serum albumin per 1 mol of the zinc ion is not particularly limited and is, for example, 1 mol or less, preferably 0.9 mol or less, more preferably 0.8 mol or less, and still more preferably 0.7 mol or less or 0.65 mol or less. A specific range of the content of the serum albumin per 1 mol of the zinc ion is, for example, 0.35 to 1 mol or 0.35 to 0.9 mol, preferably 0.4 to 0.8 mol, and more preferably 0.5 to 0.7 mol or 0.55 to 0.65 mol.

### Forms of Zinc Ion and Serum Albumin

In the first intravenous pharmaceutical composition of the present invention, the zinc ions and the serum albumin form a conjugate. Specifically, some or all of the zinc ions can form a conjugate with the serum albumin. In the present invention, the term "zinc ion" connotes both free zinc ions and zinc ions forming a conjugate with serum albumin. In addition, the amount of zinc ions refers to the total amount of free zinc ions and zinc ions forming a conjugate with serum albumin. When the serum albumin is human serum albumin, the conjugate is considered to be in a mode in which the zinc ions are bound between His67 and Asn99 of domain I and His247 and Asp249 of domain II in the human serum albumin.

### Other Components

In addition to the above components, the first intravenous pharmaceutical composition of the present invention may include other base materials and/or additives that can be used for intravenous pharmaceutical compositions. Typical examples of such bases include water, and examples of the additives include tonicity agents (typically sodium chloride) and pH adjusters.

Although the cases are not excluded in which the first intravenous pharmaceutical composition of the present invention includes, in addition to the above components, a nutrient that can be used for intravenous pharmaceutical compositions, it is preferable that the intravenous pharmaceutical composition does not include a nutrient. Examples of such a nutrient include carbohydrates (glucose, fructose, xylitol, and the like), amino acids, and vitamins.

Although the cases are not excluded in which the first intravenous pharmaceutical composition of the present invention includes, in addition to the above components, a salt that can be used for intravenous pharmaceutical compositions, it is preferable that the intravenous pharmaceutical composition does not include a salt other than a water-soluble zinc salt and sodium chloride.

### Formulation Forms and the Like

The first intravenous pharmaceutical composition of the present invention is not particularly limited as long as it can be used in the form of an aqueous intravenous pharmaceutical composition (that is, in the form of an aqueous solution of the intravenous pharmaceutical composition) at time of use.

For example, examples of the formulation form of the first intravenous pharmaceutical composition of the present invention include an aqueous intravenous pharmaceutical composition which is a form that does not require dilution or dissolution at time of use (that is, a form of an aqueous solution of the intravenous pharmaceutical composition; straight type).

Other examples of the formulation form of the first intravenous pharmaceutical composition of the present invention include a concentrated aqueous intravenous pharmaceutical composition which is a form that requires dilution with water or an aqueous solution (for example, physiological saline) at time of use (that is, a form of a concentrated aqueous solution of the intravenous pharmaceutical composition; concentrated type).

Yet other examples of the formulation form of the first intravenous pharmaceutical composition of the present invention include a solid intravenous pharmaceutical composition which is a form that requires dissolution in water or an aqueous solution (for example, physiological saline) at time of use (that is, a dry form of the intravenous pharmaceutical composition; dry type). Specific examples of the dry form include freeze-dried products and spray-dried products.

Examples of the product classification of the first intravenous pharmaceutical composition of the present invention include injections and infusions, and injections are preferable.

### Uses

The first intravenous pharmaceutical composition of the present invention is useful for hypoalbuminemia that is a condition requiring administration of zinc. The first intravenous pharmaceutical composition of the present invention is applied for the purpose of supplementing zinc for hypoalbuminemia that is a condition requiring administration of zinc. Specific examples of hypoalbuminemia include hypoalbuminemic hypozincemia, and more specifically, the examples include liver diseases such as hepatitis B, hepatitis C, alcoholic fatty liver, non-alcoholic fatty liver, and liver cirrhosis; diabetes, kidney diseases such as nephrotic syndrome, connective tissue disease, malignant tumors, undernutrition, inflammatory bowel disease, acute pancreatitis, hypertensive disorders of pregnancy, hypoalbuminemia associated with severe burns, sepsis, or the like, and drug-induced hypoalbuminemia.

Hypoalbuminemia that is a condition requiring administration of zinc may be accompanied by hyperammonemia. The first intravenous pharmaceutical composition of the present invention can also be applied for the purpose of treating hyperammonemia for a condition accompanied by hyperammonemia.

The animal species to which the first intravenous pharmaceutical composition of the present invention is applied is not particularly limited as long as it has albumin as a serum protein, and preferable examples thereof include mammals such as humans, cows, horses, cats, and dogs.

### Dosage and Administration

The administration of the first intravenous pharmaceutical composition of the present invention is not limited as long as the pharmaceutical composition is intravenously injected, and the pharmaceutical composition may be administered by injection or infusion. Among these administration methods, the administration by injection is preferable.

The dose of the first intravenous pharmaceutical composition of the present invention can be set according to the severity of hypozincemia, animal species, age, and the like, and is, for example, 0.05 to 10 mg/kg/administration, preferably 0.1 to 5 mg/kg/administration, more preferably 0.3 to 3 mg/kg/administration, still more preferably 0.5 to 1.5 mg/kg/administration, and even more preferably 0.6 to 1.2 mg/kg/administration, 0.6 to 0.8 mg/kg/administration, or 0.8 to 1.2 mg/kg/administration in terms of the zinc ion amount for a human.

The first intravenous pharmaceutical composition of the present invention is preferably repeatedly administered multiple times. The number of times the intravenous pharmaceutical composition is administered in the repeated administration is not particularly limited, and examples thereof include 2 times or more, 3 times or more, 4 times or more, 5 times or more, 6 times or more, 7 times or more, 8 times or more, 9 times or more, and 10 times or more. The upper limit of the number of times the intravenous pharmaceutical composition is administered in the repeated administration is not particularly limited, and examples thereof include 20 times or less, 15 times or less, 12 times or less, 10 times or less, 9 times or less, 8 times or less, 7 times or less, 6 times or less, and 5 times or less. Examples of the specific range of the number of times the intravenous pharmaceutical composition is administered in the repeated administration include 2 to 20 times, 3 to 15 times, 4 to 12 times, 5 to 12 times, 6 to 12 times, 7 to 12 times, 8 to 12 times, 9 to 12 times, 10 to 12 times, 4 to 10 times, 4 to 9 times, 4 to 8 times, 4 to 7 times, 4 to 6 times, and 4 or 5 times. The frequency of the administration in the repeated administration is not particularly limited, and is, for example, once every 2 to 14 days, preferably once every 3 to 12 days, more preferably once every 4 to 10 days, still more preferably once every 5 to 9 days, even more preferably once every 6 to 8 days, and particularly preferably once every 7 days.

### Production Method

The first intravenous pharmaceutical composition of the present invention is produced by any method by which the zinc ion and serum albumin can form a conjugate in the composition. Specifically, the first intravenous pharmaceutical composition can be obtained by dissolving a water-soluble zinc salt, serum albumin, and other components mixed in as necessary in water or physiological saline, stirring the mixture at room temperature for 10 minutes or more, preferably 60 minutes or more, and then performing concentration and/or drying in accordance with the formulation form as necessary.

### 2. Second Intravenous Pharmaceutical Composition

A second intravenous pharmaceutical composition of the present invention includes a zinc ion. Hereinafter, the second intravenous pharmaceutical composition of the present invention will be described in detail.

### Source of Zinc Ion

As the source of the zinc ion, a water-soluble zinc salt can be used. The water-soluble zinc salt is not particularly limited as long as it is a pharmaceutically acceptable salt. Examples of the water-soluble zinc salt include salts of inorganic acids such as hydrochloric acid, hydrobromic acid, phosphoric acid, and sulfuric acid; and salts of organic acids such as acetic acid, citric acid, tartaric acid, lactic acid, succinic acid, fumaric acid, maleic acid, and methanesulfonic acid. The water-soluble zinc salt may be an anhydrous form or a hydrated form.

As the water-soluble zinc salt in the second intravenous pharmaceutical composition of the present invention, one of the above salts may be used singly, or two or more thereof may be used in combination. Among the above salts, salts of inorganic acid salts are preferable, a sulfate (that is, zinc sulfate) is more preferable, and zinc sulfate heptahydrate is still more preferable from the viewpoint of further improving the zinc supplementation efficiency.

The content of the zinc ion may be appropriately set according to the severity of iron overload and/or the required degree of iron reduction effect, and/or the necessity of dilution and dissolution at time of use, and the like. For example, the zinc ion concentration in the intravenous pharmaceutical composition at time of use is, for example, 0.1 mM or more, preferably 0.5 mM or more, more preferably 0.7 mM or more, still more preferably 0.8 mM or more, and even more preferably 0.9 mM or more. The upper limit of the zinc concentration is not particularly limited and is, for example, 5 mM or less, preferably 4 mM or less, more preferably 3 mM or less, still more preferably 2 mM or less, even more preferably 1.5 mM or less, and yet even more preferably 1.2 mM or less. A specific range of the zinc ion concentration in the intravenous pharmaceutical composition at time of use is, for example, 0.1 to 5 mM or 0.1 to 4 mM, preferably 0.5 to 3 mM, more preferably 0.7 to 2 mM, still more preferably 0.8 to 1.5 mM, and even more preferably 0.9 to 1.2 mM.

### Serum Albumin

The second intravenous pharmaceutical composition of the present invention may or may not include serum albumin. For example, when the subject to which the second intravenous pharmaceutical composition of the present invention is applied has iron overload accompanied by hypoalbuminemia, it is preferable that the pharmaceutical composition includes serum albumin from the viewpoint of excellent iron reduction effect.

When the second intravenous pharmaceutical composition of the present invention includes serum albumin, the origin of the serum albumin is not particularly limited as long as it is common to the animal species to which the intravenous pharmaceutical composition is to be administered.

The content of the serum albumin may be appropriately set according to the severity of iron overload and/or the required degree of iron reduction effect, and the content of the serum albumin per 1 mol of the zinc ion is, for example, 0.35 mol or more, preferably 0.4 mol or more, and more preferably 0.5 mol or more or 0.55 mol or more. The upper limit of the content of the serum albumin per 1 mol of the zinc ion is not particularly limited and is, for example, 1 mol or less, preferably 0.9 mol or less, more preferably 0.8 mol or less, and still more preferably 0.7 mol or less or 0.65 mol or less. A specific range of the content of the serum albumin per 1 mol of the zinc ion is, for example, 0.35 to 0.9 mol, preferably 0.4 to 0.8 mol, and more preferably 0.5 to 0.7 mol or 0.55 to 0.65 mol.

### Forms of Zinc Ion and Serum Albumin

When the second intravenous pharmaceutical composition of the present invention includes serum albumin, the zinc ions and the serum albumin form a conjugate. Specifically, some or all of the zinc ions can form a conjugate with the serum albumin. In the present invention, the term "zinc ion" connotes both free zinc ions and zinc ions forming a conjugate with serum albumin. In addition, the amount of zinc ions refers to the total amount of free zinc ions and zinc ions forming a conjugate with serum albumin. When the serum albumin is human serum albumin, the conjugate is considered to be in a mode in which the zinc ions are bound between His67 and Asn99 of domain I and His247 and Asp249 of domain II in the human serum albumin.

### Other Components

In addition to the above components, the second intravenous pharmaceutical composition of the present invention may include other base materials and/or additives that can be used for intravenous pharmaceutical compositions. Typical examples of such bases include water, and examples of the additives include tonicity agents (typically sodium chloride) and pH adjusters.

Although the cases are not excluded in which the second intravenous pharmaceutical composition of the present invention includes, in addition to the above components, a nutrient that can be used for intravenous pharmaceutical compositions, it is preferable that the intravenous pharmaceutical composition does not include a nutrient. Examples of such a nutrient include carbohydrates (glucose, fructose, xylitol, and the like), amino acids, and vitamins.

Although the cases are not excluded in which the second intravenous pharmaceutical composition of the present invention includes, in addition to the above components, a salt that can be used for intravenous pharmaceutical compositions, it is preferable that the intravenous pharmaceutical composition does not include a salt other than a water-soluble zinc salt and sodium chloride.

### Formulation Forms and the Like

The second intravenous pharmaceutical composition of the present invention is not particularly limited as long as it can be used in the form of an aqueous intravenous pharmaceutical composition (that is, in the form of an aqueous solution of the intravenous pharmaceutical composition) at time of use.

For example, examples of the formulation form of the second intravenous pharmaceutical composition of the present invention include an aqueous intravenous pharmaceutical composition which is a form that does not require dilution or dissolution at time of use (that is, a form of an aqueous solution of the intravenous pharmaceutical composition; straight type).

Other examples of the formulation form of the second intravenous pharmaceutical composition of the present invention include a concentrated aqueous intravenous pharmaceutical composition which is a form that requires dilution with water or an aqueous solution (for example, physiological saline) at time of use (that is, a form of a concentrated aqueous solution of the intravenous pharmaceutical composition; concentrated type).

Yet other examples of the formulation form of the second intravenous pharmaceutical composition of the present invention include a solid intravenous pharmaceutical composition which is a form that requires dissolution in water or an aqueous solution (for example, physiological saline) at time of use (that is, a dry form of the intravenous pharmaceutical composition; dry type). Specific examples of the dry form include freeze-dried products and spray-dried products.

Examples of the product classification of the second intravenous pharmaceutical composition of the present invention include injections and infusions, and injections are preferable.

### Uses

The second intravenous pharmaceutical composition of the present invention is applied for iron overload. That is, the second intravenous pharmaceutical composition of the present invention treats iron overload by reducing iron in the body of a patient with iron overload.

Specific examples of iron overload include iron overload secondary to transfusion and the like, iron overload caused by overdose of iron-containing tablets, injections, and the like, hereditary iron overload, and alcohol dependence. Iron overload may also be accompanied by a complication. Examples of the complication include liver cirrhosis, hypoalbuminemia, hyperammonemia, diabetes, and heart failure. Specific examples of hypoalbuminemia include liver diseases such as hepatitis B, hepatitis C, alcoholic fatty liver, non-alcoholic fatty liver, and liver cirrhosis; diabetes, kidney diseases such as nephrotic syndrome, connective tissue disease, malignant tumors, undernutrition, inflammatory bowel disease, acute pancreatitis, hypertensive disorders of pregnancy, hypoalbuminemia associated with severe burns, sepsis, or the like, and drug-induced hypoalbuminemia.

In addition, since the second intravenous pharmaceutical composition of the present invention includes water-soluble zinc as an active ingredient, iron overload may be accompanied by a condition requiring administration of zinc. The condition requiring the administration of zinc is as described in the section of "Uses" in "1. First Intravenous Pharmaceutical Composition" above.

The animal species to which the second intravenous pharmaceutical composition of the present invention is applied is not particularly limited as long as it has albumin as a serum protein, and preferable examples thereof include mammals such as humans, cows, horses, cats, and dogs.

### Dosage and Administration

The administration of the second intravenous pharmaceutical composition of the present invention is not limited as long as the pharmaceutical composition is intravenously injected, and the pharmaceutical composition may be administered by injection or infusion. Among these administration methods, the administration by injection is preferable.

The dose of the second intravenous pharmaceutical composition of the present invention can be set according to the severity of iron overload, animal species, age, and the like, and is, for example, 0.05 to 10 mg/kg/administration, preferably 0.1 to 5 mg/kg/administration, more preferably 0.3 to 3 mg/kg/administration, still more preferably 0.5 to 1.5 mg/kg/administration, and even more preferably 0.6 to 1.2 mg/kg/administration, 0.6 to 0.8 mg/kg/administration, or 0.8 to 1.2 mg/kg/administration in terms of the zinc ion amount for a human.

The second intravenous pharmaceutical composition of the present invention is preferably repeatedly administered multiple times. The number of times the intravenous pharmaceutical composition is administered in the repeated administration is not particularly limited, and examples thereof include 2 times or more, 3 times or more, 4 times or more, 5 times or more, 6 times or more, 7 times or more, 8 times or more, 9 times or more, and 10 times or more. The upper limit of the number of times the intravenous pharmaceutical composition is administered in the repeated administration is not particularly limited, and examples thereof include 20 times or less, 15 times or less, 12 times or less, 10 times or less, 9 times or less, 8 times or less, 7 times or less, 6 times or less, and 5 times or less. Examples of the specific range of the number of times the intravenous pharmaceutical composition is administered in the repeated administration include 2 to 20 times, 3 to 15 times, 4 to 12 times, 5 to 12 times, 6 to 12 times, 7 to 12 times, 8 to 12 times, 9 to 12 times, 10 to 12 times, 4 to 10 times, 4 to 9 times, 4 to 8 times, 4 to 7 times, 4 to 6 times, and 4 or 5 times. The frequency of the administration in the repeated administration is not particularly limited, and is, for example, once every 2 to 14 days, preferably once every 3 to 12 days, more preferably once every 4 to 10 days, still more preferably once every 5 to 9 days, even more preferably once every 6 to 8 days, and particularly preferably once every 7 days.

### Production Method

The second intravenous pharmaceutical composition of the present invention can be obtained, for example, by dissolving a water-soluble zinc salt, serum albumin mixed in as necessary, and/or other components mixed in as necessary in water or physiological saline, stirring the mixture at room temperature for 10 minutes or more, preferably 60 minutes or more, and then performing concentration and/or drying in accordance with the formulation form as necessary.

### Examples

Hereinafter, the present invention will be described more specifically with reference to Examples, but the present invention is not limited thereto.

### Test Example 1: First Intravenous Pharmaceutical Composition

### Test Example 1-1

### [1] Materials Used

Albumin derived from human serum (FUJIFILM Wako Pure Chemical Corporation)
Zinc sulfate heptahydrate (FUJIFILM Wako Pure Chemical Corporation)
Physiological saline (Otsuka Pharmaceutical Factory, Inc.)
Dialysis cassette (material: regenerated cellulose, molecular weight cut-off: 10 K, Thermo Scientific Inc., product number (catalog number): 66455)

### [2] Preparation of Intravenous Solutions

Intravenous solutions having the compositions of Comparative Example 1-1 and Examples 1-1a to 1-1f in Table 1 were prepared by dissolving zinc sulfate heptahydrate in physiological saline at a concentration of 1.0 mM, further mixing in human serum albumin to 0 µM, 100 µM, 200 µM, 300 µM, 400 µM, 500 µM, or 600 µM (n = 3 for each concentration), and mixing the mixture at room temperature for 10 minutes. The pH of Example 1-1f at 25°C was about 6.9.

**[Table 1]**

| | Zinc sulfate heptahydrate | Human serum albumin |
|---|---|---|
| Comparative Example 1-1 | 1.0 mM | 0 µM |
| Example 1-1a | 1.0 mM | 100 µM |
| Example 1-1b | 1.0 mM | 200 µM |
| Example 1-1c | 1.0 mM | 300 µM |
| Example 1-1d | 1.0 mM | 400 µM |
| Example 1-1e | 1.0 mM | 500 µM |
| Example 1-1f | 1.0 mM | 600 µM |

### [3] Measurement of Concentration of Zinc Ions Bound to Albumin

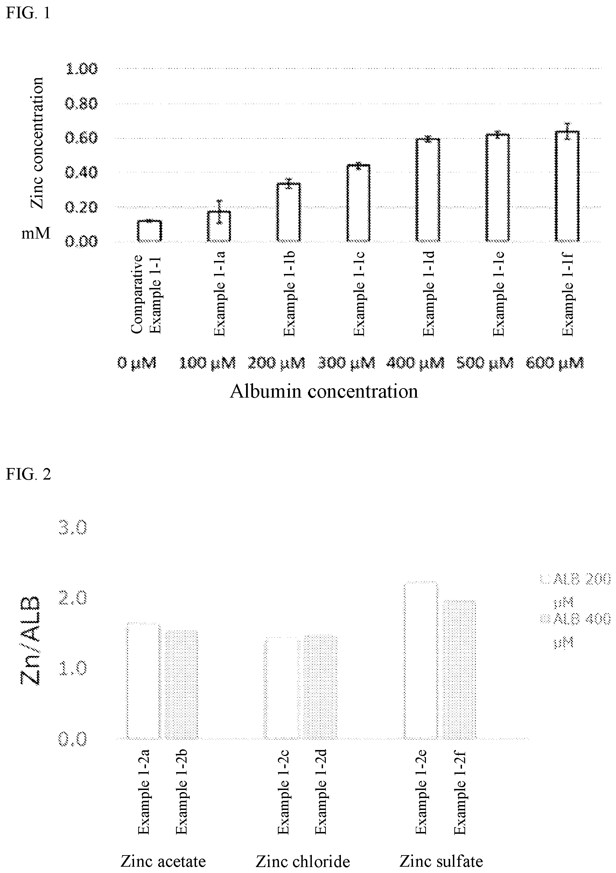

The cassette was immersed in ultrapure water for 1 to 2 minutes to be hydrophilized. Each intravenous solution was collected in a syringe in a volume of 3 mL and injected through the port of the hydrophilized cassette using a 18G injection needle. After attaching a float to the cassette, the cassette was floated in about 1000 mL of ultrapure water, and dialysis was performed at room temperature for 2 hours. Then, the cassette was floated again in about 1000 mL of fresh ultrapure water, and dialysis was performed at room temperature for 2 hours. The cassette was then floated in about 1000 mL of fresh ultrapure water, and overnight dialysis was performed once at 4°C. After completion of the dialysis, the post-dialysis intravenous solution was collected through the port of the cassette into which the needle was not inserted using a 18G injection needle and syringe. Measurement of zinc (direct method: 5-Br-PAPS) in the post-dialysis intravenous solution was performed using an automatic analyzer 7180 (Hitachi High-Tech Corporation). The results are shown in Fig. 1.

As shown in Fig. 1, the amount of zinc bound to albumin increased in a manner dependent on the concentration of albumin that was mixed in, and when the amount of albumin that was mixed in was 400 µM or more, the amount of zinc bound to the albumin plateaued. That is, it was found that when albumin is mixed in to a concentration of 400 µM or more with respect to 1 mM zinc (the albumin concentration being 0.4 mol or more per 1 mol of zinc), albumin capable of binding to zinc was saturated.

### Test Example 1-2

Intravenous solutions of Examples 1-2a to 1-2f composed of water-soluble zinc salts and albumin mixed in physiological saline were prepared with the compositions as shown in Table 2.

| | Zinc acetate dihydrate | Zinc chloride | Zinc sulfate heptahydrate | Human serum albumin |
|---|---|---|---|---|
| Example 1-2a | 1.0 mM | - | - | 200 µM |
| Example 1-2b | 1.0 mM | - | - | 400 µM |
| Example 1-2c | - | 1.0 mM | - | 200 µM |
| Example 1-2d | - | 1.0 mM | - | 400 µM |
| Example 1-2e | - | - | 1.0 mM | 200 µM |
| Example 1-2f | - | - | 1.0 mM | 400 µM |

The prepared intravenous solutions were subjected to the method described in [3] of Test Example 1-1 to perform the measurement of zinc (direct method: 5-Br-PAPS) and albumin (BCG method) in the post-dialysis intravenous solutions. A value (Zn/ALB) was obtained by dividing the measured zinc concentration (Zn) by the measured albumin concentration (ALB). The larger the value of Zn/ALB, the larger the binding strength of the zinc ions to albumin. The results are shown in Fig. 2.

As a result, the binding of the zinc ions to albumin was observed in all Examples, and as shown in Fig. 2, it was found that the binding strength of the zinc ions to albumin was larger in Examples 1-2e and 1-2f than in Examples 1-2a to 1-2d. Since albumin is suggested to be a carrier for hepatocytes to acquire zinc in the body, it is possible to say that the larger the binding strength of a zinc salt to albumin, the higher the zinc supplying effect to the liver. Therefore, it can be reasonably inferred that the sulfate has a higher zinc supplying effect to the liver than the acetate and chloride of zinc.

### Test Example 1-3

### [1] Materials Used

Albumin derived from human serum (FUJIFILM Wako Pure Chemical Corporation)
Zinc sulfate heptahydrate (FUJIFILM Wako Pure Chemical Corporation)
Physiological saline (Otsuka Pharmaceutical Factory, Inc.)

### [2] Preparation of Test Intravenous Solutions

### <Example>

Albumin derived from human serum and zinc sulfate heptahydrate were mixed into physiological saline at 600 µM and 1.0 mM, respectively (the albumin concentration being 0.6 mol per 1 mol of zinc), and the mixture was stirred with a stirrer at room temperature for about 30 minutes to prepare a test intravenous solution of Example 1-3 (also described as "Zn-Alb").

### <Comparative Example>

Zinc sulfate heptahydrate was mixed into physiological saline at 1.0 mM, and the mixture was stirred with a stirrer at room temperature for about 30 minutes to prepare a test intravenous solution of Comparative Example 3 (also described as "Zn").

**[Table 3]**

| | Zinc sulfate heptahydrate | Human serum albumin | Administration method | Dose of zinc (single administration) | Dose of zinc/ administration (repeated administration) |
|---|---|---|---|---|---|
| Comparative Example 1-3 (Zn) | 1.0 mM | 0 µM | Intravenous injection | 0.325 mg/kg | 0.65 mg/kg |
| Example 1-3 (Zn-Alb) | 1.0 mM | 600 µM | Intravenous injection | 0.325 mg/kg | 0.65 mg/kg |

### [3] Animals Used and Rearing Conditions

Female (8 to 9 weeks old) and male (6 to 7 weeks old) rats of the NAR/Slc strain (Japan SLC, Inc.) were used. This strain is a mutant rat that exhibits analbuminemia due to autosomal recessive inheritance, and is used as a model of hypoalbuminemic patients.

These rats were fed with a zinc-deficient diet (Oriental Yeast Co., Ltd.) for 7 weeks to prepare a model of hypoalbuminemia and hypozincemia. During the feeding, the rats had ad libitum access to ultrapure water (replaced twice a week) from a water absorption bottle.

### [4] Administration of Test Intravenous Solutions

### [4-1] Single Administration

The administration site was the tail vein, the administration was performed a single time, and the dose was 5 mL/kg. This dose is 0.325 mg/kg in terms of the zinc amount and is equivalent to the dose of 16.3 mg for a human weighing 50 kg. The day of the single administration was counted as day 0 of administration (Day 0), and the day after the single administration was counted as one day after administration (Day 1).

### [4-2] Repeated Administration

The administration site was the tail vein, the administration was performed 8 times (once/week), and the dose was 10 mL/kg. This dose is 0.65 mg/kg/administration in terms of the zinc amount and is equivalent to the dose of 32.6 mg/administration for a human weighing 50 kg. The day of the first repeated administration was counted as day 28 of administration (Day 28), and the day after the first repeated administration was counted as 29 days after administration (Day 29).

### [4-3] Administration method

The animals were immobilized using an immobilization device. A sterilized indwelling needle (24 gauge, Surflo F & F, Terumo Corporation) was inserted and indwelled in the tail vein without anesthesia. For administration, the test substance was collected in a syringe barrel and administered via a catheter using a syringe. The administration rate was about 2.0 mL/kg/min.

### [5] Measurement of Serum Zinc Concentration and Results

### [5-1] Case of Single Administration

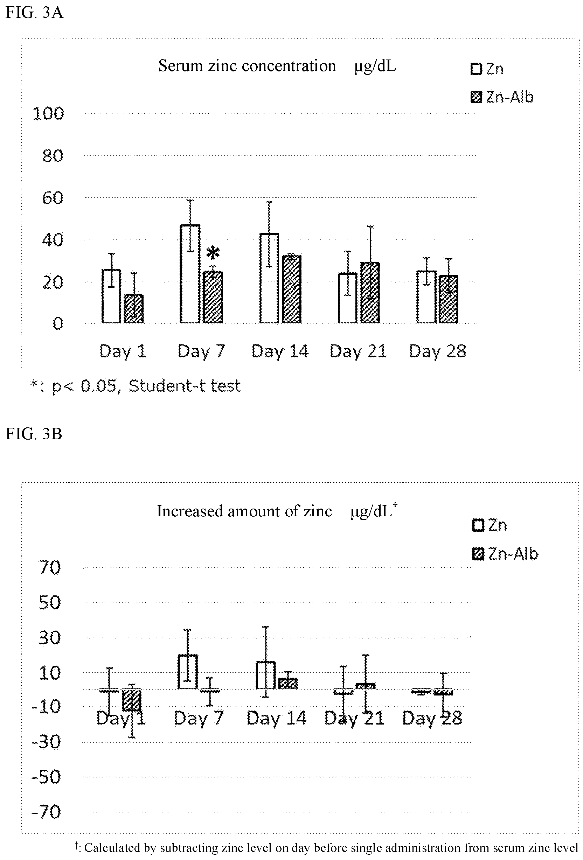

The measurement was performed in the afternoon on the day before the single administration and on Day 1, Day 7, Day 14, Day 21, and Day 28 after the single administration. The animals were immobilized manually, and 300 µL of blood was collected from the jugular vein without anesthesia. The blood was transferred to a tube (Microtainer blood collection tube, Nippon Becton Dickinson Company, Ltd.) containing a coagulation accelerator/serum-separating agent. The mixture was left to stand at room temperature for 30 to 60 minutes and then centrifuged with a floor standing refrigerated centrifuge S700FR (3000 rpm, 10 min, 4°C) to obtain serum.
The concentration of zinc in the serum was measured using an automatic analyzer 7180 (Hitachi High-Tech Corporation). The results are shown in Fig. 3A. Furthermore, the increased amount of zinc was calculated by subtracting the zinc concentration (zinc level) on the day before the single administration from the zinc concentrations (zinc levels) on Day 1, Day 7, Day 14, Day 21, and Day 28 after the single administration. The results are shown in Fig. 3B.

### [5-2] Case of Repeated Administration

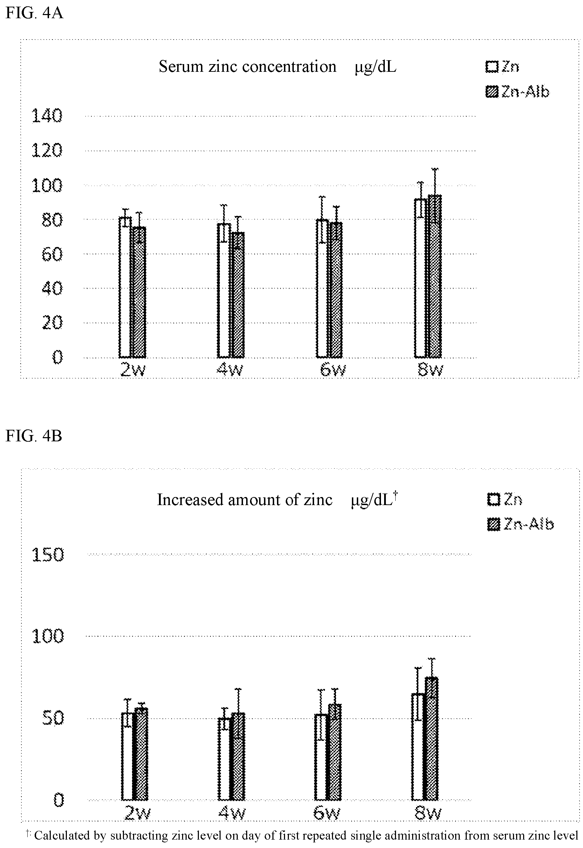

Measurement was performed in the afternoon on Day 14 (2 W), Day 28 (4 W), Day 42 (6 W), and Day 56 (8 W) after the first repeated administration and before the repeated administration on the same days (that is, the seventh day after the previous administration). The animals were immobilized manually, and 300 µL of blood was collected from the jugular vein without anesthesia. The blood was transferred to a tube (Microtainer blood collection tube, Nippon Becton Dickinson Company, Ltd.) containing a coagulation accelerator/serum-separating agent. The mixture was left to stand at room temperature for 30 to 60 minutes and then centrifuged with a floor standing refrigerated centrifuge S700FR (3000 rpm, 10 min, 4°C) to obtain serum.
The concentration of zinc in the serum was measured using an automatic analyzer 7180 (Hitachi High-Tech Corporation). The results are shown in Fig. 4A. Furthermore, the increased amount of zinc was calculated by subtracting the zinc concentration (zinc level) on the day of the first repeated administration from the zinc concentrations (zinc levels) on Day 14 (2 W), Day 28 (4 W), Day 42 (6 W), and Day 56 (8 W) after the first repeated administration. The results are shown in Fig. 4B.

### [5-3] Results

In the single administration, as shown in the results of "Day 7" in Figs. 3A and B, the serum zinc concentration was observed to be transiently lower in the case of administering the test intravenous solution of Example 1-3 (Zn-Alb) as compared with the case of administering the test intravenous solution of Comparative Example 1-3 (Zn). In the dynamics of zinc in the body, zinc that has been absorbed is first carried to the liver and then distributed throughout the body (Practice guideline for zinc deficiency. 2018. The Japanese Society of Clinical Nutrition, 2018. p. 1-46.). Thus, the low serum zinc concentration observed on "Day 7" in Figs. 3A and B reasonably indicates a fluctuation reflecting the high bioabsorbable property of the novel zinc formulation.

In addition, in the repeated administration (measurement results on the seventh day after the previous administration), as shown in Fig. 4B, a stable increase in zinc was observed in the case of administering the test intravenous solution of Example 1-3 (Zn-Alb) as compared with the case of administering the test intravenous solution of Comparative Example 1-3 (Zn), and as observed in Fig. 4A, after 8 weeks, the blood zinc concentration in the case of administering the test intravenous solution of Example 1-3 (Zn-Alb) became higher than that in the case of administering the test intravenous solution of Comparative Example 1-3 (Zn). In the results of the repeated administration (measurement results on the seventh day after the previous administration), the increase in the serum zinc level reasonably indicates that the zinc absorbed into the liver by the previous administration was redistributed in the blood.

### Test Example 1-4

### [1] Materials Used and Preparation of Test Intravenous Solutions

Using the materials used in Test Example 1-3, intravenous solutions of Comparative Example 1-4 and Examples 1-4a to 1-4c composed of the zinc source and albumin mixed in physiological saline were prepared with the compositions as shown in Table 4.

**[Table 4]**

| | Zinc sulfate heptahydrate | Human serum albumin | Administration method | Dose of zinc/ administration (repeated administration) |
|---|---|---|---|---|
| Comparative Example 1-4 (Zn) | 1.0 mM | 0 µM | Intravenous injection | 0.975 mg/kg |
| Example 1-4a (Zn-Alb 200) | 1.0 mM | 200 µM | Intravenous injection | 0.975 mg/kg |
| Example 1-4b (Zn-Alb 400) | 1.0 mM | 400 µM | Intravenous injection | 0.975 mg/kg |
| Example 1-4c (Zn-Alb 600) | 1.0 mM | 600 µM | Intravenous injection | 0.975 mg/kg |

### [2] Animals Used and Rearing Conditions

The same animals and rearing conditions were used as in Test Example 1-3.

### [3] Administration of Test Intravenous Solutions

### [3-1] Repeated Administration

The administration site was the tail vein, the administration was performed 10 times (once/week), and the dose was 15 mL/kg. This dose is 0.975 mg/kg/administration in terms of the zinc amount, is equivalent to the dose of 48.9 mg/administration for a human weighing 50 kg, and is about 14 times the daily amount of zinc in the ASPEN parenteral nutrition recommendations, which is 3 to 4 mg. The first administration to the tenth administration are described as Dose 1 to Dose 10, respectively.

### [3-2] Administration method

The animals were immobilized using an immobilization device. A sterilized indwelling needle (24 gauge, Surflo F & F, Terumo Corporation) was inserted and indwelled in the tail vein without anesthesia. For administration, the test substance was collected in a syringe barrel and administered via a catheter using a syringe. The administration rate was about 4.0 mL/kg/min.

### [4] Blood Biochemical Tests and Results

The tests were performed in the morning on the day after each repeated administration (that is, the first day after the previous administration). The animals were immobilized manually, and 500 µL of blood was collected from the jugular vein without anesthesia. The blood was transferred to a tube (Microtainer blood collection tube, Nippon Becton Dickinson Company, Ltd.) containing a coagulation accelerator/serum-separating agent. The mixture was left to stand at room temperature for 30 to 60 minutes and then centrifuged with a refrigerated centrifuge (3000 rpm, 10 min, 4°C), and the obtained serum was used for the test. The serum was subjected to measurement using an automatic analyzer BM6050. The ammonia reduction amount (Serum NH₃ red) was calculated as a reduction amount relative to the day before the first administration using the levels of ammonia measured at each blood collection and the day before the first administration.

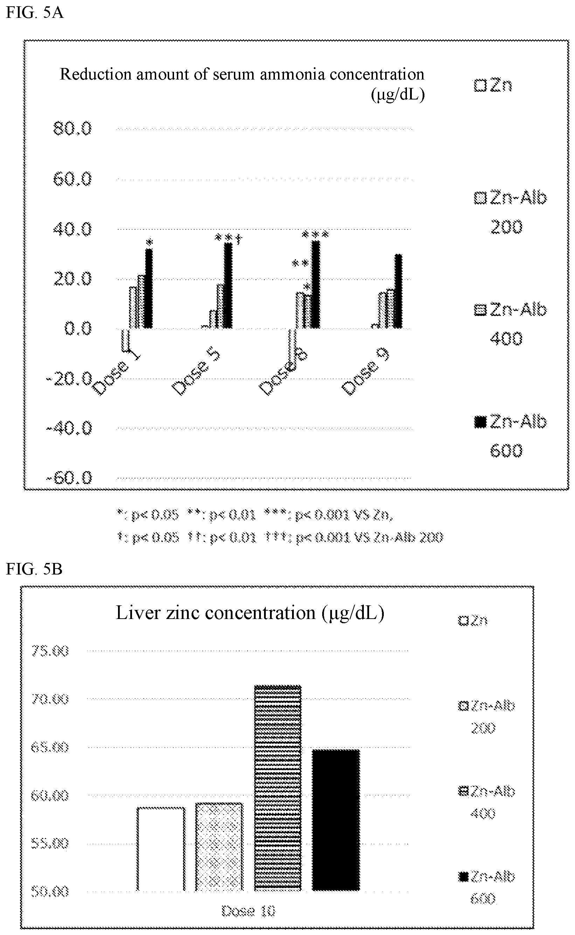

The average levels of zinc and albumin in each group on the day before the first administration were 65 µg/dL and 1.3 g/dL or less, respectively, and it was confirmed that the present model was established as a hypoalbuminemic hypozincemia animal model. In addition, the ammonia level exceeded the average level of 135 µg/dL in each group, also confirming that the present model reflected the disease state of hyperammonemia. The results of measuring ammonia by the repeated administration in Comparative Example 1-4 (Zn) and Examples 1-4a to 1-4c (Zn-Alb 200, 400, and 600) are shown in Fig. 5A.

As shown in Fig. 5A, the amount of ammonia was significantly reduced (the extent of reduction in the amount of ammonia was significantly increased) by the administration of the intravenous solutions of Examples 1-4a to 1-4c (Zn-Alb 200, 400, and 600) as compared with the administration of the intravenous solution of Comparative Example 1-4 (Zn), thus suggesting the treatment effect for hyperammonemia accompanying hypoalbuminemic hypozincemia. In the urea cycle, which is the center of ammonia metabolism in the body, a zinc enzyme ornithine transcarbamylase (OTC) functions in hepatocytes. The hyperammonemia observed as a background value of the model animals was considered to be a result of inhibition of the enzyme activity of OTC due to zinc deficiency, and the ammonia metabolizing effect observed in a post-administration albumin concentration-dependent manner was considered to be a result of efficient transfer of zinc to the liver by albumin, which was the result that complemented the high zinc supplementation effect of the intravenous solutions of Examples 1-4a to 1-4c (Zn-Alb 200, 400, and 600).

### [5] Measurement of Liver Zinc Concentration and Results

### [5-1] Measurement Method

After the blood collection was completed, the animals were subjected to exsanguination. Then, the livers were collected, and tissues cryopreserved at -80°C were used as samples. To 0.5 g of each sample that had been thawed, 10 mL of 0.1 N hydrochloric acid was added. After homogenization, the mixture was allowed to stand for 30 minutes, and the supernatant was centrifuged (10,000 rpm, 10 min, 4°C). Using the obtained supernatant as an assay specimen, absorbance at 560 nm was measured using Metallo Assay Zinc Measurement LS (Metallogenics Co., Ltd.) and a microplate reader, and the zinc concentration was calculated.

### [5-2] Results

The results are shown in Fig. 5B. As shown in Fig. 5B, the amount of zinc in the liver was increased by the administration of the intravenous solutions of Examples 1-4a to 1-4c (Zn-Alb 200, 400, and 600) as compared with the administration of the intravenous solution of Comparative Example 1-4 (Zn). Since zinc binds to albumin in the blood to be carried to the organs throughout the body, the increase in the amount of zinc reflected the high tissue migration effect of the zinc formulation containing albumin as a zinc carrier, that is, the high zinc supplementation effect to the liver. According to Fig. 5B, the storage amount of zinc in the liver is the largest in Example 1-4b (Zn-Alb 400). However, in view of the zinc supplementation results shown by the ammonia metabolizing effect in Fig. 5A of the present test example and the effect of redistributing zinc in the blood in Figs. 4A and 4B of Test Example 1-3, Example 1-4c (Zn-Alb 600) can be evaluated as the formulation that is most excellent in the zinc supplementation effect that allows zinc to be stored in the liver and to stably exist in the blood as well by redistribution.

### Test Example 2: Second Intravenous Pharmaceutical Composition

### Test Example 2-1 (Preliminary Test on Effect of Removing Iron in the Body)

### [1] Materials Used and Preparation of Test Samples

Test samples of Examples 2-1a and 2-1b and Reference Example were prepared by mixing the following components in physiological saline at the contents in the table, and stirring the mixture with a stirrer at room temperature for about 10 minutes.

**[Table 5]**

| | Zinc sulfate heptahydrate | Human serum albumin | Deferasirox | Administration method | Dose/administration (repeated administration) |
|---|---|---|---|---|---|
| Example 2-1a (Zn) | 1.0 mM | 0 µM | - | Intravenous injection | 15 mL/kg (dose of intravenous solution) |
| | | | | | 0.975 mg/kg (dose of zinc) |
| Example 2-1b (Zn-Alb) | 1.0 mM | 600 µM | - | Intravenous injection | 15 mL/kg (dose of intravenous solution) |
| | | | | | 0.975 mg/kg (dose of zinc) |
| Reference Example (Defe) | - | - | 10 mg/mL | Oral administration | 18 mg/kg |

### [2] Animals Used and Rearing Conditions

Crl: 18 male (6 to 7 weeks old) rats (The Jackson Laboratory Japan, Inc.) of the (CD) SD strain were used.

During the test period, the rats had ad libitum access to standard feed CRF-1 (Oriental Yeast Co., Ltd.) and tap water through a sterilization filtration device.

### [3] Administration of Test Samples

In Examples 2-1a and 2-1b, tail vein administration (administration rate: 4.0 mL/kg/min) was performed, and in Reference Example, oral administration (administration by sonde) was performed. The administration was performed 7 times, and the administration frequency was once/day. The doses are as indicated in Table 5, and the doses in Examples 2-1a and 2-1b are 0.975 mg/kg in terms of the zinc amount and are equivalent to the dose of 48.75 mg for a human weighing 50 kg. The dose of deferasirox (Novartis Pharma K.K. "Jadenu", therapeutic agent for iron overload) in Reference Example is the upper limit of the clinical dose.

### [4] Blood Biochemical Tests and Results

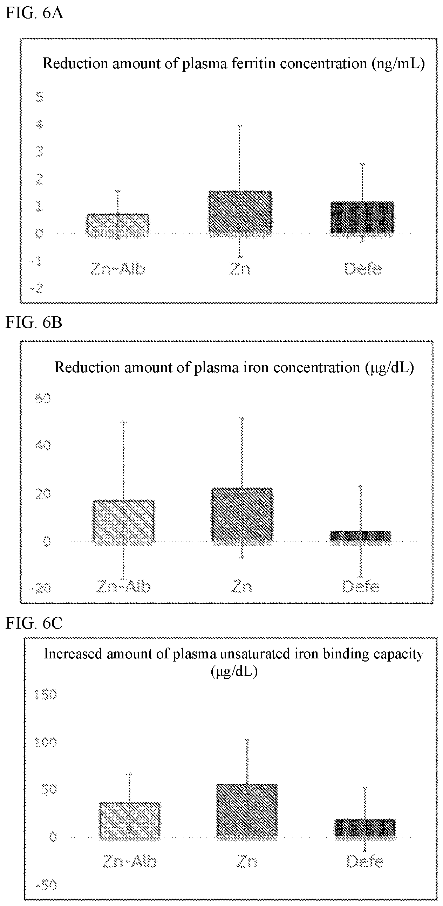

The blood biochemical tests were performed on the grouping day and the day after the final administration for all the cases. From each of the tail vein and the caudal vena cava, 300 µL or more was collected for the blood biochemical tests. All blood collection was performed in the state of being fasted for 16 hours or more from the previous day. The collected blood was quickly transferred to a microtube containing heparin and the blood and heparin were mixed. Plasma was collected by centrifugation (1900 × g, 10 min, 4°C), and iron (bathophenanthroline direct method), unsaturated iron binding capacity (UIBC) (nitroso-PSAP method), and ferritin (ELISA method) in the plasma were measured. As for the amounts of iron and ferritin, the reduction amounts were derived by subtracting the measured value on the day after the final administration from the measured value on the grouping day. The results are shown in Figs. 6A and 6B. As for the unsaturated iron binding capacity, the increased amount was derived by subtracting the measured value on the grouping day from the measured value on the day after the final administration. The results are shown in Fig. 6C.

The ferritin reducing effect, the iron reducing effect, and the UIBC increasing effect of both intravenous solutions Zn (Example 2-1a) and Zn-Alb (Example 2-1b) shown in Figs. 6A to 6C were shown to be at the same levels as those of deferasirox (Reference Example), which is a known therapeutic agent for iron overload.

### Test Example 2-2 (Effect of Removing Iron in Bodies of Iron-Loaded Rats)

### [1] Materials Used and Preparation of Intravenous Solutions

Intravenous solutions of Comparative Example 2-2 and Examples 2-2a and 2-2b were prepared by mixing the following components in physiological saline at the contents in the table.

**[Table 6]**

| | Zinc sulfate heptahydrate | Human serum albumin | Administration method | Dose/administration (repeated administration) |
|---|---|---|---|---|
| Comparative Example 2-2 (Saline) | 0 µM | 0 µM | Intravenous injection | 50 mL/kg (dose of intravenous solution) |
| Example 2-2a (Zn-Sul) | 1.0 mM | 0 µM | Intravenous injection | 50 mL/kg (dose of intravenous solution) |
| | | | | 3.25 mg/kg (dose of zinc) |
| Example 2-2b (Zn-Alb) | 1.0 mM | 600 µM | Intravenous injection | 50 mL/kg (dose of intravenous solution) |
| | | | | 3.25 mg/kg (dose of zinc) |

### [2] Animals Used and Rearing Conditions

Crl: 24 male (6 to 7 weeks old) rats (The Jackson Laboratory Japan, Inc.) of the (CD) SD strain were used. Iron loading was performed without anesthesia by single subcutaneous administration of a dextran iron solution (Sigma-Aldrich Japan G.K.) at two locations on the back (dose: 400 mg/kg) using an injection needle and a syringe barrel, thereby preparing an iron overload (hemochromatosis) model.

During the test period, the rats had ad libitum access to standard feed CRF-1 (Oriental Yeast Co., Ltd.) and tap water through a sterilization filtration device.

### [3] Administration of Intravenous Solutions

From the third week after the iron loading, Comparative Example 2-2 and Examples 2-2a and 2-2b were administered via the tail vein (administration rate: 4.0 mL/kg/min). The administration was performed 14 times, and the administration frequency was once/day. The dose was about 3 times the dose in Test Example 2-1, as indicated in Table 6. This dose is 3.25 mg/kg in terms of the zinc amount and is equivalent to the dose of 162.5 mg for a human weighing 50 kg.

### [4] Blood Biochemical Tests and Results

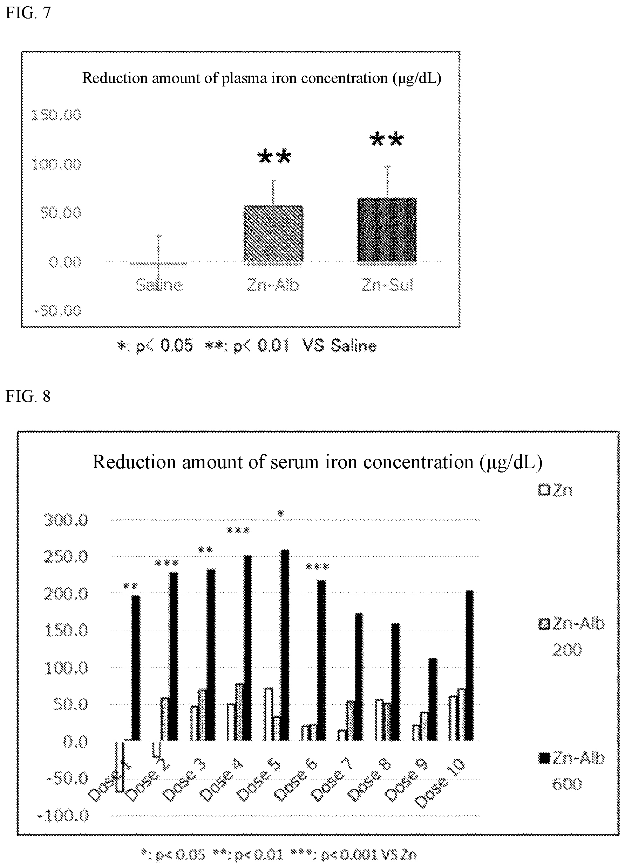

The blood biochemical test was performed three weeks after the iron loading and on the day after the final administration for all the cases. From each of the jugular vein and the caudal vena cava, 500 µL or more was collected for the blood biochemical test. All blood collection was performed in the state of being fasted for 16 hours or more from the previous day. In the same manner as in Test Example 2-1, iron in plasma was measured, and the iron reduction amount was derived by subtracting the measured value on the day after the final administration from the measured value three weeks after the iron loading. The results are shown in Fig. 7.

As shown in Fig. 7, both of the intravenous solutions Zn-Sul (Example 2-2a) and Zn-Alb (Example 2-2b) were confirmed to have an iron reducing effect on the iron overload model. Based on the comparison results between deferasirox (Reference Example) and Zn (Example 2-1a) and Zn-Alb (Example 2-1b) in Test Example 2-1, it is inferred that any of the intravenous solutions Zn-Sul (Example 2-2a) and Zn-Alb (Example 2-2b) has the treatment effect for iron overload equivalent to that of deferasirox.

### Test Example 2-3

### [1] Materials Used and Preparation of Test Intravenous Solutions

Using the materials used in Test Example 1-3, intravenous solutions of Examples 2-3a to 2-3c composed of the zinc source and albumin mixed in physiological saline were prepared with the compositions as shown in Table 7.

**[Table 7]**

| | Zinc sulfate heptahydrate | Human serum albumin | Administration method | Dose of zinc/administration (repeated administration) |
|---|---|---|---|---|
| Example 2-3a (Zn) | 1.0 mM | 0 µM | Intravenous injection | 0.975 mg/kg |
| Example 2-3b (Zn-Alb 200) | 1.0 mM | 200 µM | Intravenous injection | 0.975 mg/kg |
| Example 2-3c (Zn-Alb 600) | 1.0 mM | 600 µM | Intravenous injection | 0.975 mg/kg |

### [2] Animals Used and Rearing Conditions

The same animals and rearing conditions were used as in Test Example 1-3.

### [3] Administration of Test Intravenous Solutions

### [3-1] Repeated Administration

The administration site was the tail vein, the administration was performed 10 times (once/week), and the dose was 15 mL/kg. This dose is 0.975 mg/kg/administration in terms of the zinc amount, is equivalent to the dose of 48.9 mg/administration for a human weighing 50 kg, and is about 14 times the daily amount of zinc in the ASPEN parenteral nutrition recommendations, which is 3 to 4 mg. The first administration to the tenth administration are described as Dose 1 to Dose 10, respectively.

### [3-2] Administration method

The animals were immobilized using an immobilization device. A sterilized indwelling needle (24 gauge, Surflo F & F, Terumo Corporation) was inserted and indwelled in the tail vein without anesthesia. For administration, the test substance was collected in a syringe barrel and administered via a catheter using a syringe. The administration rate was about 4.0 mL/kg/min.

### [4] Blood Biochemical Tests and Results

The tests were performed in the morning on the day after each repeated administration (that is, the first day after the previous administration). The animals were immobilized manually, and 500 µL of blood was collected from the jugular vein without anesthesia. The blood was transferred to a tube (Microtainer blood collection tube, Nippon Becton Dickinson Company, Ltd.) containing a coagulation accelerator/serum-separating agent. The mixture was left to stand at room temperature for 30 to 60 minutes and then centrifuged with a refrigerated centrifuge (3000 rpm, 10 min, 4°C), and the obtained serum was used for the test. The serum was subjected to measurement using an automatic analyzer BM6050. The iron reduction amount (Serum Fe red) was calculated as a reduction amount relative to the day before the first administration using the levels of iron measured at each blood collection and the day before the first administration.

The average levels of zinc and albumin in each group on the day before the first administration were 65 µg/dL and 1.3 g/dL or less, respectively, and it was confirmed that the present model was established as a hypoalbuminemic hypozincemia animal model. **In** addition, the ammonia level exceeded the average level of 135 µg/dL in each group, also confirming that the present model reflected the disease state of hyperammonemia. Furthermore, since high iron measurement values were observed, it was confirmed that the present model also reflected the disease state of iron overload. The results of measuring iron affected by the repeated administration in Example 2-3a (Zn), Example 2-3b (Zn-Alb 200), and Example 2-3c (Zn-Alb 600) are shown in Fig. 8.

As shown in Fig. 8, it was confirmed that the amounts of iron were reduced by the repeated administration in Example 2-3a (Zn), Example 2-3b (Zn-Alb 200), and Example 2-3c (Zn-Alb 600). **In** addition, as compared with the administration of the intravenous solution of Example 2-3a (Zn), the reduction amounts of iron tended to be greater with the intravenous solutions of Example 2-3b (Zn-Alb 200) and Example 2-3c (Zn-Alb 600), and the reduction was particularly significant with the intravenous solution of Example 2-3c (Zn-Alb 600). In other words, it was shown that the iron clearance effect for iron overload accompanying hypoalbuminemic hypozincemia was higher with the intravenous solution obtained by further mixing albumin with water-soluble zinc in a manner dependent on the ratio of albumin mixed in.

## Claims

1. An intravenous pharmaceutical composition comprising:
a zinc ion; and
serum albumin.

2. The intravenous pharmaceutical composition according to claim 1, wherein the zinc ion and the serum albumin form a conjugate.

3. The intravenous pharmaceutical composition according to claim 1, wherein a source of the zinc ion is a water-soluble zinc salt.

4. The intravenous pharmaceutical composition according to claim 3, wherein the water-soluble zinc salt is zinc sulfate.

5. The intravenous pharmaceutical composition according to claim 1, wherein the content of the serum albumin per 1 mol of the zinc ion is 0.35 mol or more.

6. The intravenous pharmaceutical composition according to claim 1, wherein the content of the zinc ion at time of use is 0.1 mM or more.

7. The intravenous pharmaceutical composition according to claim 1, wherein the intravenous pharmaceutical composition is free of a carbohydrate.

8. The intravenous pharmaceutical composition according to claim 1, wherein the intravenous pharmaceutical composition is an injection.

9. The intravenous pharmaceutical composition according to claim 1, wherein the intravenous pharmaceutical composition is applied for hypoalbuminemia that is a condition requiring administration of zinc.

10. The intravenous pharmaceutical composition according to claim 9, wherein the condition is hypoalbuminemic hypozincemia.

11. The intravenous pharmaceutical composition according to claim 9, wherein the intravenous pharmaceutical composition is applied for hyperammonemia.

12. An intravenous pharmaceutical composition comprising a zinc ion,
wherein the intravenous pharmaceutical composition is used for treatment of iron overload.

13. The intravenous pharmaceutical composition according to claim 12, wherein a source of the zinc ion is a water-soluble zinc salt.

14. The intravenous pharmaceutical composition according to claim 13, wherein the water-soluble zinc salt is zinc sulfate.

15. The intravenous pharmaceutical composition according to claim 12, wherein the content of the zinc ion at time of use is 0.1 mM or more.

16. The intravenous pharmaceutical composition according to claim 12, wherein the intravenous pharmaceutical composition is an injection.

17. The intravenous pharmaceutical composition according to claim 12, further comprising serum albumin.

18. The intravenous pharmaceutical composition according to claim 17, wherein the zinc ion and the serum albumin form a conjugate.

19. The intravenous pharmaceutical composition according to claim 17, wherein the content of the serum albumin per 1 mol of the zinc ion is 0.35 mol or more.

20. The intravenous pharmaceutical composition according to claim 17, wherein the iron overload is accompanied by hypoalbuminemia.

21. The intravenous pharmaceutical composition according to claim 20, wherein the hypoalbuminemia is hypoalbuminemic hypozincemia.

22. The intravenous pharmaceutical composition according to claim 12, wherein the iron overload is accompanied by hyperammonemia.

23. The intravenous pharmaceutical composition according to claim 12, wherein the intravenous pharmaceutical composition is free of a carbohydrate.
